(19) Europäisches Patentamt
European Patent Office
Office européen des brevets

(11) Veröffentlichungsnummer : **0 588 762 A1**

(12) # EUROPÄISCHE PATENTANMELDUNG

(21) Anmeldenummer : **93810595.4**

(51) Int. Cl.[5] : **A61K 31/505**

(22) Anmeldetag : **23.08.93**

(30) Priorität : **31.08.92 CH 2729/92**

(43) Veröffentlichungstag der Anmeldung : **23.03.94 Patentblatt 94/12**

(84) Benannte Vertragsstaaten : **AT BE CH DE DK ES FR GB GR IE IT LI LU NL PT SE**

(71) Anmelder : **CIBA-GEIGY AG**
**Klybeckstrasse 141**
**CH-4002 Basel (CH)**

(72) Erfinder : **Zimmermann, Jürg Dr.**
**Weidenpark 1**
**CH-4313 Möhlin (CH)**
Erfinder : **Caravatti, Giorgio Dr.**
**Baselmattweg 145/c**
**CH-4123 Allschwil (CH)**

(54) **Verwendung von Pyrimidinderivaten als Proteinkinase C-Inhibitoren und Antitumormittel.**

(57) Die Erfindung betrifft die Verwendung bekannter N-Phenyl-2-pyrimidinamin-derivate der Formel I zur Hemmung der Proteinkinase C in Warmblütern, d.h. z.B. als Antitumormittel.

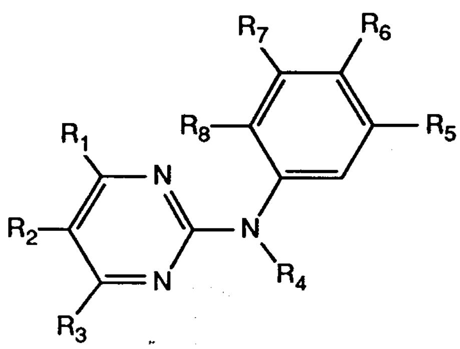

(I)

Die Substituenten in Formel I haben die in Anspruch 1 angegebenen Bedeutungen.

EP 0 588 762 A1

Jouve, 18, rue Saint-Denis, 75001 PARIS

Die Erfindung betrifft die Verwendung bekannter N-Phenyl-2-pyrimidinamin-derivate zur Hemmung der Proteinkinase C und/oder als Antitumormittel sowie zur Herstellung von pharmazeutischen Präparaten zur Anwendung als Hemmer der Proteinkinase C und/oder als Antitumormittel in Warmblütern.

Die Europäische Patentanmeldung mit der Anmeldenummer 87100277.0, welche am 26. 8. 1987 unter der Veröffentlichungsnummer 0233461 veröffentlicht wurde, und die dem US Patent Nr. 4,876,252 teilweise äquivalent ist, beschreibt N-Phenyl-2-pyrimidinamin-derivate, deren Herstellung und ihre Verwendung als Antiasthmatika und Antiallergika aufgrund der Hemmung der Histaminausschüttung, sowie ihre Verwendung bei Entzündungen und Diabetes.

Erfindungsgemäss wurde nun gefunden, dass ein Teil der in der EP-A-0233461 beschriebenen N-Phenyl-2-pyrimidinamin-derivate selektiv das Enzym Proteinkinase C hemmen.

Die von Phospholipiden und Calcium abhängige Proteinkinase C kommt innerhalb der Zelle in mehreren Spezies (Verteilung der Spezies gewebespezifisch) vor und beteiligt sich an verschiedenen fundamentalen Vorgängen, wie Signalübertragung, Proliferation und Differenzierung, sowie auch Ausschüttung von Hormonen und Neurotransmittern. Die Aktivierung dieses Enzyms erfolgt entweder durch eine über Rezeptoren vermittelte Hydrolyse von Phospholipiden der Zellmembran oder durch eine direkte Interaktion mit gewissen Tumor-fördernden Wirkstoffen. Zelluläre Funktionen, die mit Hilfe der Proteinkinase C gesteuert werden, können durch die Modulation der Enzymaktivität von Proteinkinase C beeinflusst werden.

Die Erfindung betrifft die Verwendung von N-Phenyl-2-pyrimidinamin-derivaten der Formel I,

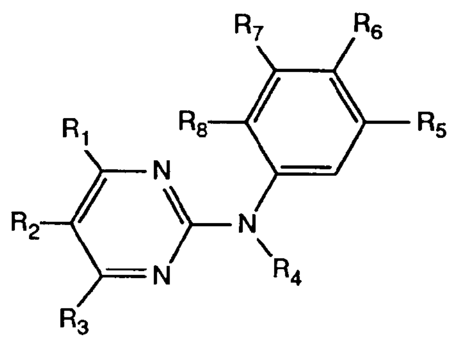

(I)

worin $R_1$ Wasserstoff oder $C_1$-$C_3$-Alkyl bedeutet, $R_2$ Wasserstoff oder $C_1$-$C_3$-Alkyl bedeutet, $R_3$ 2-Pyridyl, 3-Pyridyl, 4-Pyridyl, 2-Methyl-3-pyridyl, 4-Methyl-3-pyridyl, 2-Furyl, 5-Methyl-2-furyl, 2,5-Dimethyl-3-furyl, 2-Thienyl, 3-Thienyl, 5-Methyl-2-thienyl, 2-Phenothiazinyl, 4-Pyrazinyl, 2-Benzofuryl, N-Oxido-2-pyridyl, N-Oxido-3-pyridyl, N-Oxido-4-pyridyl, 1H-Indol-2-yl, 1H-Indol-3-yl, 1-Methyl- 1H-pyrrol-2-yl, 4-Quinolinyl, 1-Methyl-pyridinium-4-yl-iodid, Dimethylamino-phenyl oder N-Acetyl-N-methyl-aminophenyl bedeutet, $R_4$ Wasserstoff, $C_1$-$C_3$-Alkyl, den Rest -CO-CO-O-$C_2H_5$ oder N,N-Dimethylaminoethyl bedeutet, mindestens einer der Reste $R_5$, $R_5$ , $R_7$ und $R_5$ $C_1$-$C_6$-Alkyl, $C_1$-$C_3$-Alkoxy, Chlor, Brom, Iod, Trifluormethyl, Hydroxy, Phenyl, Amino, Mono-($C_1$-$C_3$-alkyl)-amino, Di-($C_1$-$C_3$-alkyl)-amino, $C_2$-$C_4$-Alkanoyl, Propenyloxy, Carboxy, Carboxy-methoxy, Ethoxycarbonyl-methoxy, Sulfanilamido, N,N-Di-($C_1$-$C_3$-alkyl)-sulfanilamido, N-Methyl-piperazinyl, Piperidinyl, 1H-Imidazol-1-yl, 1H-Triazol-1-yl, 1H-Benzimidazol-2-yl, 1-Naphthyl, Cyclopentyl, 3,4-Dimethyl-benzyl oder einen Rest einer der Formeln:

-$CO_2$R, -NH-C(=O)-R, -N(R)-C(=O)-R, -O-$(CH_2)_n$-N(R)-R, -C(=O)-NH-$(CH_2)_n$-N(R)-R, -CH($CH_3$)-NH-CHO, -C($CH_3$)=N-OH, -C($CH_3$)=N-O-$CH_3$, -C($CH_3$)-$NH_2$, -NH-$CH_2$-C(=O)-N(R)-R,

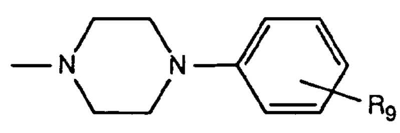
,

-$(CH_2)_m$-$R_{10}$, -X-$(CH_2)_m$-$R_{10}$ oder

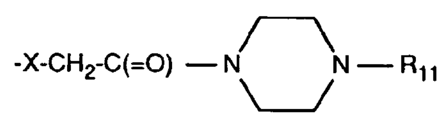

bedeutet, worin R für $C_1$-$C_3$-Alkyl steht, X für Sauerstoff oder Schwefel steht, m für 1, 2 oder 3 steht, n für 2

oder 3 steht, $R_9$ für Wasserstoff, $C_1$-$C_3$-Alkyl, $C_1$-$C_3$-Alkoxy, Chlor, Brom, Iod oder Trifluormethyl steht, $R_{10}$ für 1H-Imidazol-1-yl oder Morpholinyl steht und $R_{11}$ für $C_1$-$C_3$-Alkyl oder unsubstituiertes oder durch $C_1$-$C_3$-Alkyl, Halogen oder Trifluormethyl monosubstituiertes Phenyl steht, und die übrigen der Reste $R_5$, $R_6$, $R_7$ und $R_6$ Wasserstoff bedeuten, oder von pharmazeutisch verwendbaren Salzen von solchen Verbindungen mit mindestens einer salzbildenden Gruppe zur Hemmung der Proteinkinase C und zur Herstellung von pharmazeutischen Präparaten zur Anwendung als Hemmer der Proteinkinase C in Warmblütern. Die erfindungsgemässe Verwendung als Hemmer der Proteinkinase C erstreckt sich nicht auf die vorbeschriebenen Verwendungen gegen Asthma, Allergien, Entzündung und Diabetes, selbst wenn die Wirkung gegen diese Krankheiten ursächlich auf die Hemmung der Proteinkinase C zurückzuführen sein sollte.

Die Verbindungen der Formel I und ihre Herstellung sind in der EP-A-0233461 und im US Patent 4,876,252 beschrieben.

Salzbildende Gruppen in einer Verbindung der Formel I sind Gruppen oder Reste mit basischen oder sauren Eigenschaften. Verbindungen mit mindestens einer basischen Gruppe oder mindestens einem basischen Rest, z. B. einer freien Aminogruppe oder einem Pyridylrest können Säureadditionssalze bilden, z.B. mit anorganischen Säuren, wie Salzsäure, Schwefelsäure oder einer Phosphorsäure, oder mit geeigneten organischen Carbon- oder Sulfonsäuren, z.B. aliphatischen Mono- oder Dicarbonsäuren, wie Trifluoressigsäure, Essigsäure, Propionsäure, Glykolsäure, Bernsteinsäure, Maleinsäure, Fumarsäure, Hydroxymaleinsäure, Aepfelsäure, Weinsäure, Zitronensäure, Oxalsäure oder Aminosäuren, wie Arginin oder Lysin, aromatischen Carbonsäuren, wie Benzoesäure, 2-Phenoxy-benzoesäure, 2-Acetoxy-benzoesäure, Salicylsäure, 4-Aminosalicylsäure, aromatisch-aliphatischen Carbonsäuren, wie Mandelsäure oder Zimtsäure, heteroaromatischen Carbonsäuren, wie Nicotinsäure oder Isonicotinsäure, aliphatischen Sulfonsäuren, wie Methan-, Ethan- oder 2-Hydroxy-ethan-sulfonsäure, oder aromatischen Sulfonsäuren, z.B. Benzol-, p-Toluol- oder Naphthalin-2-sulfonsäure. Bei Anwesenheit von mehreren basischen Gruppen können Mono- oder Polysäureadditionssalze gebildet werden.

Verbindungen der Formel I mit sauren Gruppen, z.B. einer freien Carboxylgruppe, können Metall- oder Ammoniumsalze, wie Alkalimetall- oder Erdalkalimetallsalze bilden, z.B. Natrium-, Kalium-, Magnesium- oder Calciumsalze oder Ammoniumsalze mit Ammoniak oder geeigeten organischen Aminen, wie tertiären Monoaminen, z.B. Triethylamin oder Tri-(2-hydroxyethyl)-amin, oder heterocyclischen Basen, z.B. N-Ethyl-piperidin oder N,N'-Dimethyl-piperazin.

Wie erfindungsgemäss gefunden wurde, besitzen die Verbindungen der Formel I bisher noch unbekannte, wertvolle pharmakologische Eigenschaften, z.B. hemmen sie selektiv das Enzym Proteinkinase C.

Zur Bestimmung der Proteinkinase-C-Hemmwirkung verwendet man Proteinkinase C aus Schweinehirn. Die Bestimmung der Proteinkinase-C-Hemmwirkung der Verbindungen der Formel I erfolgt nach der Methodik von D. Fabbro et al. wie beschrieben im Beispiel 2. In diesem Test hemmen die Verbindungen der Formel I die Proteinkinase C bereits bei einer Konzentration $IC_{50}$ zwischen etwa 1 und 30 $\mu$Mol/Liter.

Demgegenüber hemmen die Verbindungen der Formel I andere Enzyme, z.B. Proteinkinase A und Protein-Phosphorylase-Kinase, erst bei einer weitaus, z.B 100fach höheren Konzentration. Dies zeigt die Selektivität der Verbindungen der Formel I.

Aufgrund ihrer Hemmwirkung gegenüber Proteinkinase C können die Verbindungen der Formel I und ihre pharmazeutisch verwendbaren Salze als tumorhemmende, immunomodulierende und antibakterielle Wirkstoffe, ferner als Mittel gegen Atherosklerose, die Immunschwächekrankheit AIDS, sowie Krankheiten des kardiovaskulären Systems und des zentralen Nervensystems, verwendet werden.

Wie bereits aufgrund der obengeschilderten Hemmwirkung auf Proteinkinase C erwartet werden kann, weisen die Verbindungen der Formel I antiproliferative Eigenschaften auf, die sich unter anderem in dem im Beispiel 1 beschriebenen Versuch direkt demonstrieren lassen. Dabei wird die Hemmwirkung der Verbindungen der Formel I auf das Wachstum von menschlichen T24 Blasenkarzinomzellen bestimmt. Die so ermittelten $IC_{50}$-Werte liegen für die Verbindungen der Formel I zwischen etwa 1 und 10 $\mu$Mol/Liter.

Auch in vivo lassen sich die antiproliferativen Eigenschaften demonstrieren, z.B. wie beschrieben im Beispiel 3. In dem im Beispiel 3 beschriebenen Versuch bewirken die Verbindungen der Formel I nach peroraler oder intraperitonealer Verabreichung eine Reduktion des Tumorvolumens auf etwa 35-70 % des Tumorvolumens bei mit Placebo behandelten Kontrolltieren.

Aufgrund der beschriebenen Eigenschaften können die Verbindungen der Formel I insbesondere als tumorhemmende Wirkstoffe verwendet werden, z.B zur Therapie von Tumoren der Blase. Die Erfindung betrifft diese Verwendung als Antitumormittel unabhängig davon, ob die Antitumorwirkung ursächlich auf die Hemmung der Proteinkinase C zurückgeht oder nicht, und unabhängig davon, ob nachgewiesen werden kann, dass die gefundene Antitumorwirkung ursächlich auf die Hemmung der Proteinkinase C zurückgeht.

Wenn die Verbindungen der Formel I bei der Krebstherapie in Kombination mit anderen Chemotherapeutika verwendet werden, verhindern sie die Bildung von Resistenz (multidrug resistance) oder heben eine bereits gegenüber den anderen Chemotherapeutika vorhandene Resistenz auf. Ausserdem kommen die Verbindungen der Formel I für die oben für Proteinkinase C Modulatoren genannten weiteren Anwendungen in Betracht und können insbesondere zur Behandlung von Krankheiten verwendet werden, die auf eine Hemmung der Proteinkinase C ansprechen. Die Erfindung betrifft die Verwendung der Verbindungen der Formel I für die obengenannten Zwecke, mit Ausnahme der vorbeschriebenen Verwendung gegen Asthma, Allergien, Entzündung und Diabetes, und zur Herstellung von pharmazeutischen Präparaten zur Anwendung für diese Zwecke.

Bevorzugt verwendet man Verbindungen der Formel I, worin mindestens zwei der Reste $R_5$, $R_6$ und $R_5$ je für Wasserstoff stehen, insbesondere solche Verbindungen, worin $R_5$, $R_6$ und $R_8$ je für Wasserstoff stehen und die übrigen Substituenten jeweils die obengenannten Bedeutungen haben, und pharmazeutisch verwendbare Salze von solchen Verbindungen mit mindestens einer salzbildenden Gruppe.

Hauptsächlich verwendet man Verbindungen der Formel I, worin $R_1$ , $R_2$, $R_4$, $R_5$, $R_6$ und $R_5$ je für Wasserstoff stehen, $R_3$ 2-Pyridyl, 3-Pyridyl, 4-Pyridyl, 2-Methyl-3-pyridyl, 4-Methyl-3-pyridyl, 2-Furyl, 5-Methyl-2-furyl, 2,5-Dimethyl-3-furyl, 2-Thienyl, 3-Thienyl, 5-Methyl-2-thienyl, 2-Phenothiazinyl, 4-Pyrazinyl, 2-Benzofuryl, N-Oxido-2-pyridyl, N-Oxido-3-pyridyl, N-Oxido-4-pyridyl, 1H-Indol-2-yl, 1H-Indol-3-yl, 1-Methyl-1H-pyrrol-2-yl, 4-Quinolinyl, 1-Methyl-pyridinium-4-yl-iodid, Dimethylaminophenyl oder N-Acetyl-N-methyl-amino-phenyl bedeutet, und $R_7$ $C_1$-$C_6$-Alkyl, $C_1$-$C_3$-Alkoxy, Chlor, Brom, Iod, Trifluormethyl, Hydroxy, Phenyl, Amino, Mono-($C_1$-$C_3$-alkyl)-amino, Di-($C_1$-$C_3$-alkyl)-amino, $C_2$-$C_4$-Alkanoyl, Propenyloxy, Carboxy, Carboxymethoxy, Ethoxycarbonyl-methoxy, Sulfanilamido, N,N-Di-($C_1$-$C_3$-alkyl)-sulfanilamido, N-Methyl-piperazinyl, Piperidinyl, 1H-Imidazol-1-yl, 1H-Triazol-1-yl, 1H-Benzimidazol-2-yl, 1-Naphthyl, Cyclopentyl, 3,4-Dimethyl-benzyl oder einen Rest einer der Formeln:

$-CO_2R$, $-NH-C(=O)-R$, $-N(R)-C(=O)-R$, $-O-(CH_2)_n-N(R)-R$, $-C(=O)-NH-(CH_2)_n-N(R)-R$, $-CH(CH_3)-NH-CHO$, $-C(CH_3)=N-OH$, $-C(CH_3)=N-O-CH_3$, $-C(CH_3)-NH_2$, $-NH-CH_2-C(=O)-N(R)-R$,

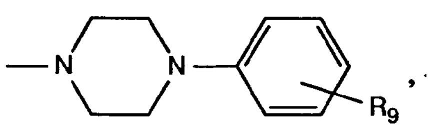 ,

$-(CH_2)_m-R_{10}$, $-X-(CH_2)_m-R_{10}$ oder

$-X-CH_2-C(=O)$ — 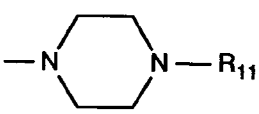

bedeutet, worin R für $C_1$-$C_3$-Alkyl steht, X für Sauerstoff oder Schwefel steht, m für 1, 2 oder 3 steht, n für 2 oder 3 steht, $R_9$ für Wasserstoff, $C_1$-$C_3$-Alkyl, $C_1$-$C_3$-Alkoxy, Chlor, Brom, Iod oder Trifluormethyl steht, $R_{10}$ für 1H-Imidazol-1-yl oder Morpholinyl steht und $R_{11}$ für $C_1$-$C_3$-Alkyl oder unsubstituiertes oder durch $C_1$-$C_3$-Alkyl, Halogen oder Trifluormethyl monosubstituiertes Phenyl steht, und pharmazeutisch verwendbare Salze von solchen Verbindungen mit mindestens einer salzbildenden Gruppe.

Besonders bevorzugt verwendet man Verbindungen der Formel I, worin $R_1$, $R_2$, $R_4$, $R_5$, $R_5$ und $R_8$ je für Wasserstoff stehen, $R_3$ 3-Pyridyl bedeutet und $R_7$ 1H-Imidazol-1-yl, Amino, Trifluormethyl, Chlor oder einen Rest der Formel $-CO_2R$ oder

$C(=O)-NH-(CH_2)_n-N(R)-R$ bedeutet, worin R je für Wasserstoff oder Methyl und n für 3 stehen, und pharmazeutisch verwendbare Säureadditionssalze von solchen Verbindungen.

Am meisten bevorzugt verwendet man die in den Beispielen genannten Verbindungen der Formel I und pharmazeutisch verwendbare Säureadditionssalze von solchen Verbindungen.

Die Erfindung betrifft auch ein Verfahren zur Behandlung von Warmblütern, die an einer Tumorerkrankung leiden, wobei man Warmblütern, die einer solchen Behandlung bedürfen, eine wirksame tumorhemmende Menge einer Verbindung der Formel I oder eines pharmazeutisch verwendbaren Salzes davon verabreicht. Die Erfindung betrifft ausserdem die Verwendung einer Verbindung der Formel I oder eines pharmazeutisch verwendbaren Salzes davon zur Hemmung der Proteinkinase C bei Warmblütern oder zur Herstellung von pharmazeutischen Präparaten zur Anwendung zur therapeutischen Behandlung des menschlichen oder tierischen Körpers. Die Erfindung betrifft auch eine Methode zur Hemmung der Proteinkinase C in Warmblütern, wobei man Warmblütern, die einer solchen Behandlung bedürfen, eine wirksame Proteinkinase C hemmende Menge einer Verbindung der Formel I verabreicht. Dabei werden an einen Warmblüter von etwa 70 kg Körpergewicht je nach Spezies, Alter, individuellem Zustand, Applikationsweise und dem jeweiligen Krankheitsbild tägliche Dosen von etwa 1-2000 mg, insbesondere 50-2000 mg, hauptsächlich 500-2000 mg, z.B. 500-1000

mg, enteral oder parenteral verabreicht.

Die Erfindung betrifft auch die Verwendung einer Verbindung der Formel I oder eines pharmazeutisch verwendbaren Salzes davon zur Herstellung pharmazeutischer Präparate zur Anwendung zur Hemmung der Proteinkinase C, z.B. zur Behandlung von Tumorerkrankungen. Die genannten pharmazeutischen Präparate enthalten eine wirksame Menge, insbesondere eine zur Prophylaxe oder Therapie einer der obengenannten Krankheiten wirksame Menge, der Aktivsubstanz zusammen mit pharmazeutisch verwendbaren Trägerstoffen, die sich zur topischen, enteralen, z.B. oralen oder rektalen, oder parenteralen Verabreichung eignen, und anorganisch oder organisch, fest oder flüssig sein können. Zur oralen Verabreichung verwendet man insbesondere Tabletten oder Gelatinekapseln, welche den Wirkstoff zusammen mit Verdünnungsmitteln, z.B. Lactose, Dextrose, Sukrose, Mannitol, Sorbitol, Cellulose und/oder Glycerin, und/oder Schmiermitteln, z.B. Kieselerde, Talk, Stearinsäure oder Salze davon, wie Magnesium- oder Calciumstearat, und/oder Polyethylenglykol, enthalten. Tabletten können ebenfalls Bindemittel, z.B. Magnesiumaluminiumsilikat, Stärken, wie Mais-, Weizen- oder Reisstärke, Gelatine, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmacksstoffe und Süssmittel enthalten. Ferner kann man die pharmakologisch wirksamen Verbindungen der vorliegenden Erfindung in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Drucks und/oder Puffer enthalten. Die vorliegenden pharmazeutischen Präparate, die, wenn erwünscht, weitere pharmakologisch wirksame Stoffe, wie Antibiotika, enthalten können, werden in an sich bekannter Weise, z.B. mittels konventioneller Misch-, Granulier-, Dragier-, Lösungs- oder Lyophilisierungsverfahren, hergestellt und enthalten von etwa 1 % bis 100 %, insbesondere von etwa 1 % bis etwa 20 %, des bzw. der Aktivstoffe.

Die nachfolgenden Beispiele illustrieren die Erfindung, ohne sie in irgendeiner Form einzuschränken.

Abkürzungen:

Verbindung A = N-(3-1H-Imidazol-1-yl-phenyl)-4-(3-pyridyl)-2-pyrimidinamin (= Verbindung der Formel I, worin $R_1$, $R_2$, $R_4$, $R_5$, $R_5$ und $R_5$ je für Wasserstoff stehen, $R_3$ 3-Pyridyl bedeutet und $R_7$ 1H-Imidazol-1-yl ist.)
Verbindung B = N-(3-Trifluormethyl-phenyl)-4-(3-pyndyl)-2-pyrimidinamin
Verbindung C = N-(3-Chlor-phenyl)-4-(3-pyridyl)-2-pyrimidinamin
Verbindung D = N-(3-Amino-phenyl)-4-(3-pyridyl)-2-pyrimidinamin
Verbindung E = N-(3-Methoxycarbonyl-phenyl)-4-(3-pyridyl)-2-pyrimidinamin
Verbindung F = N-(3-[3-Amino-propylamino-carbonyl]-phenyl)-4-(3-pyridyl)-2-pyrimidinamin

Beispiel 1: Hemmung des Wachstums von menschlichen Blasenkarzinomzellen

Menschliche T24 Blasenkarrinomzellen werden in "Eagle's minimal essential medium", dem 5 % (V/V) fötales Kälberserum zugesetzt sind, in einem befeuchteten Inkubator bei 37°C und 5 Volumenprozent $CO_2$ in der Luft inkubiert. Die Karzinomzellen (1000-1500) werden in 96-Loch-Mikrotiterplatten überimpft und über Nacht unter den obengenannten Bedingungen inkubiert. Die Testsubstanz wird in seriellen Verdünnungen am Tag 1 hinzugefügt. Die Platten werden unter den obengenannten Bedingungen 5 Tage lang inkubiert. Während dieser Zeitspanne durchlaufen die Kontrollkulturen mindestens 4 Zellteilungen. Nach der Inkubation werden die Zellen mit 3,3%iger (G/V) wässriger Glutaraldehydlösung fixiert, mit Wasser gewaschen und mit 0,05%iger (Gewicht/Volumen) wässriger Methylenblaulösung gefärbt. Nach dem Waschen wird der Farbstoff mit 3%iger (G/V) wässriger Salzsäure eluiert. Danach wird die optische Dichte (OD) pro Loch, welche der Zellanzahl direkt proportional ist, mit einem Photometer (Titertek multiskan) bei 665 nm gemessen. Die $IC_{50}$-Werte werden mit einem Computersystem unter Verwendung der Formel

$$\frac{OD_{665}\text{ (Test) minus }OD_{665}\text{ (Anfang)}}{OD_{665}\text{ (Kontrolle) minus }OD_{665}\text{ (Anfang)}} \times 100$$

errechnet. Die $IC_{50}$-Werte sind als diejenige Wirkstoffkonzentration definiert, bei der die Anzahl der Zellen pro Loch am Ende der Inkubationszeit nur 50 % der Zellanzahl in den Kontrollkulturen beträgt. In dem beschriebenen Test werden für Verbindungen der Formel I die folgenden $IC_{50}$-Werte erhalten:

| Verbindung | [μMol/Liter] |
|---|---|
| A | 2,5 |
| B | 7,4 |
| C | 5,2 |
| D | 7,2 |

Beispiel 2: Bestimmung der Proteinkinase-C-Hemmwirkung

Zur Bestimmung der Proteinkinase-C-Hemmwirkung verwendet man Proteinkinase C aus Schweinehirn, welche gemäss der von T. Uchida und C.R. Filburn in J. Biol. Chem. 259, 12311-4 (1984) beschriebenen Verfahrensweise gereinigt wird. Die Bestimmung der Proteinkinase-C-Hemmwirkung der Verbindungen der Formel I erfolgt nach der Methodik von D. Fabbro et al., Arch. Biochem. Biophys. 239, 102-111 (1985). In dem beschriebenen Test werden für Verbindungen der Formel I die folgenden $IC_{50}$-Werte erhalten:

| Verbindung | [μMol/Liter] |
|---|---|
| A | 8,8 |
| B | 2,5 |
| C | 6,3 |
| D | 28 |
| E | 26 |
| F | 28 |

Beispiel 3: Antitumoraktivität in Mäusen

Am Tag 0 transplantiert man je ein etwa 25 mg schweres Stück eines menschlichen T24 Blasenkarzinoms mittels Trokar subkutan unter operabler "Forene"-Narkose auf je eine weibliche Balb/c nude Maus (Balb/c nu/nu, Bomholdgaard, Dänemark; sechs Mäuse pro Gruppe). Am Tag 6 nach der Tumortransplantation beträgt das mittlere Tumorvolumen 120-140 $mm^3$ und man beginnt mit der Behandlung, die darin besteht, dass man einmal täglich während 15 aufeinanderfolgenden Tagen, d.h. insgesamt 15mal, 25 ml/kg der nachstehenden Formulierung peroral oder intraperitoneal verabreicht. Diese Formulierung wird folgendermassen hergestellt: 16 mg eines Wirkstoffs der Formel I werden in 0,4 ml Dimethylsulfoxid (100 %) gelöst.

Dazu fügt man 0,05 ml Tween 80 und mischt. Anschliessend fügt man 7,6 ml einer 0,9%igen wässerigen Natriumchloridlösung hinzu und mischt sofort gründlich. Diese Formulierung wird täglich frisch angesetzt. Die Kontrolltiere erhalten ein Placebo. Als Placebo verwendet man die gleiche Formulierung ohne Wirkstoff der Formel I.

24 Stunden nach der letzten Applikation wird das Tumorvolumen gemessen und das Verhältnis [%] des Tumorvolumens T/C bei den behandelten (T) und den mit Placebo behandelten Kontrolltieren (C) bestimmt, wobei das Tumorvolumen in den Kontrolltieren als 100 % definiert wird, d.h. je kleiner das Verhältnis T/C ist, desto wirksamer ist die verabreichte Formulierung.

Verwendet man als Wirkstoff der Formel I die Verbindung B, so ermittelt man bei peroraler Verabreichung von 25 bzw. 50 mg Verbindung B im obigen Versuch ein Verhältnis T/C von 68 % bzw. 53 %. Bei intraperitonealer Verabreichung von 25 bzw. 50 mg Verbindung B wird im obigen Versuch ein Verhältnis T/C von 54 % bzw. 46 % ermittelt.

Beispiel 4:

Tabletten, enthaltend 20 mg an Wirkstoff, z.B. eine der in den Beispielen 1-2 genannten Verbindungen der Formel I, werden in folgender Zusammensetzung in üblicher Weise hergestellt:

| Zusammensetzung: | |
|---|---|
| Wirkstoff | 20 mg |
| Weizenstärke | 60 mg |
| Milchzucker | 50 mg |
| Kolloidale Kieselsäure | 5 mg |
| Talk | 9 mg |
| Magnesiumstearat | 1 mg |
| | 145 mg |

Herstellung:

Der Wirkstoff wird mit einem Teil der Weizenstärke, mit Milchzucker und kolloidaler Kieselsäure gemischt und die Mischung durch ein Sieb getrieben. Ein weiterer Teil der Weizenstärke wird mit der 5fachen Menge Wasser auf dem Wasserbad verkleistert und die Pulvermischung mit diesem Kleister angeknetet, bis eine schwach plastische Masse entstanden ist.

Die plastische Masse wird durch ein Sieb von ca. 3 mm Maschenweite gedrückt, getrocknet und das erhaltene trockene Granulat nochmals durch ein Sieb getrieben. Darauf werden die restliche Weizenstärke, Talk und Magnesiumstearat zugemischt und die Mischung zu Tabletten von 145 mg Gewicht mit Bruchkerbe verpresst.

Beispiel 5:

Tabletten enthaltend 1 mg an Wirkstoff, z.B. eine der in den Beispielen 1-2 genanntenVerbindungen der Formel I, werden in folgender Zusammensetzung in üblicher Weise hergestellt:

| Zusammensetzung: | |
|---|---|
| Wirkstoff | 1 mg |
| Weizenstärke | 60 mg |
| Milchzucker | 50 mg |
| Kolloidale Kieselsäure | 5 mg |
| Talk | 9 mg |
| Magnesiumstearat | 1 mg |
| | 126 mg |

Herstellung:

Der Wirkstoff wird mit einem Teil der Weizenstärke, mit Milchzucker und kolloidaler Kieselsäure gemischt und die Mischung durch ein Sieb getrieben. Ein weiterer Teil der Weizenstärke wird mit der 5fachen Menge Wasser auf dem Wasserbad verkleistert und die Pulvermischung mit diesem Kleister angeknetet, bis eine schwach plastische Masse entstanden ist.

Die plastische Masse wird durch ein Sieb von ca. 3 mm Maschenweite gedrückt, getrocknet und das erhaltene trockene Granulat nochmals durch ein Sieb getrieben. Darauf werden die restliche Weizenstärke, Talk und Magnesiumstearat zugemischt und die Mischung zu Tabletten von 126 mg Gewicht mit Bruchkerbe verpresst.

Beispiel 6:

Kapseln, enthaltend 10 mg an Wirkstoff, z.B. eine der in den Beispielen 1-2 genannten Verbindungen der Formel I, werden wie folgt auf übliche Weise hergestellt:

| Zusammensetzung: | |
|---|---|
| Wirkstoff | 2500 mg |
| Talkum | 200 mg |
| Kolloidale Kieselsäure | 50 mg |

Herstellung:

Die aktive Substanz wird mit Talkum und kolloidaler Kieselsäure innig gemischt, das Gemisch durch ein Sieb mit 0,5 mm Maschenweite getrieben und dieses in Portionen von jeweils 11 mg in Hartgelatinekapseln geeigneter Grösse abgefüllt.

## Patentansprüche

1. Verwendung von N-Phenyl-2-pyrimidinamin-derivaten der Formel I,

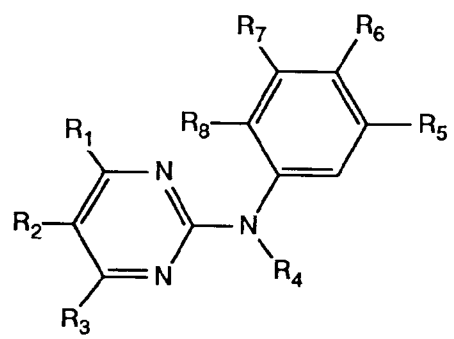

(I)

worin $R_1$ Wasserstoff oder $C_1$-$C_3$-Alkyl bedeutet, $R_2$ Wasserstoff oder $C_1$-$C_3$-Alkyl bedeutet, $R_3$ 2-Pyridyl, 3-Pyridyl, 4-Pyridyl, 2-Methyl-3-pyridyl, 4-Methyl-3-pyridyl, 2-Furyl, 5-Methyl-2-furyl, 2,5-Dimethyl-3-furyl, 2-Thienyl, 3-Thienyl, 5-Methyl-2-thienyl, 2-Phenothiazinyl, 4-Pyrazinyl, 2-Benzofuryl, N-Oxido-2-pyridyl, N-Oxido-3-pyridyl, N-Oxido-4-pyridyl, 1H-Indol-2-yl, 1H-Indol-3-yl, 1-Methyl-1H-pyrrol-2-yl, 4-Quinolinyl, 1-Methyl-pyridinium-4-yl-iodid, Dimethylamino-phenyl oder N-Acetyl-N-methyl-aminophenyl bedeutet, $R_4$ Wasserstoff, $C_1$-$C_3$-Alkyl, den Rest $-CO-CO-O-C_2H_5$ oder N,N-Dimethylaminoethyl bedeutet, mindestens einer der Reste $R_5$, $R_6$, $R_7$ und $R_8$ $C_1$-$C_6$-Alkyl, $C_1$-$C_3$-Alkoxy, Chlor, Brom, Iod, Trifluormethyl, Hydroxy, Phenyl, Amino, Mono-($C_1$-$C_3$-alkyl)-amino, Di-($C_1$-$C_3$-alkyl)-amino, $C_2$-$C_4$-Alkanoyl, Propenyloxy, Carboxy, Carboxy-methoxy, Ethoxycarbonyl-methoxy, Sulfanilamido, N,N-Di-($C_1$-$C_3$-alkyl)-sulfanilamido, N-Methyl-piperazinyl, Piperidinyl, 1H-Imidazol-1-yl, 1H-Triazol-1-yl, 1H-Benzimidazol-2-yl, 1-Naphthyl, Cyclopentyl, 3,4-Dimethyl-benzyl oder einen Rest einer der Formeln:
$-CO_2R$, $-NH-C(=O)-R$, $-N(R)-C(=O)-R$, $-O-(CH_2)_n-N(R)-R$, $-C(=O)-NH-(CH_2)_n-N(R)-R$, $-CH(CH_3)-NH-CHO$, $-C(CH_3)=N-OH$, $-C(CH_3)=N-O-CH_3$, $-C(CH_3)-NH_2$, $-NH-CH_2-C(=O)-N(R)-R$,

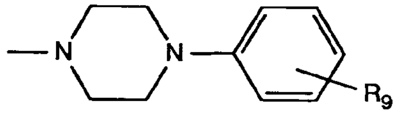,

$-(CH_2)_m-R_{10}$, $-X-(CH_2)_m-R_{10}$ oder

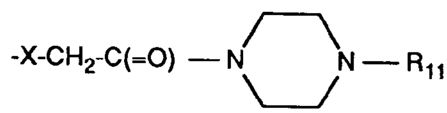

bedeutet, worin R für $C_1$-$C_3$-Alkyl steht, X für Sauerstoff oder Schwefel steht, m für 1, 2 oder 3 steht, n für 2 oder 3 steht, $R_9$ für Wasserstoff, $C_1$-$C_3$-Alkyl, $C_1$-$C_3$-Alkoxy, Chlor, Brom, Iod oder Trifluormethyl steht, $R_{10}$ für 1H-Imidazol-1-yl oder Morpholinyl steht und $R_{11}$ für $C_1$-$C_3$-Alkyl oder unsubstituiertes oder durch $C_1$-$C_3$-Alkyl, Halogen oder Trifluormethyl monosubstituiertes Phenyl steht, und die übrigen der Reste $R_5$, $R_6$, $R_7$ und $R_8$ Wasserstoff bedeuten, oder von pharmazeutisch verwendbaren Salzen von solchen Verbindungen mit mindestens einer salzbildenden Gruppe zur Herstellung von pharmazeutischen Präparaten zur Anwendung als Hemmer der Proteinkinase C in Warmblütern einschliesslich des Menschen.

2. Verwendung nach Anspruch 1 zur Herstellung von pharmazeutischen Präparaten zur Anwendung als Antitumormittel in Warmblütern einschliesslich des Menschen.

3. Verwendung nach Anspruch 1 mit Ausnahme einer etwaigen Verwendung gegen Asthma, Allergien, Entzündung und Diabetes.

4. Verwendung von N-Phenyl-2-pyrimidinamin-derivaten der Formel I,

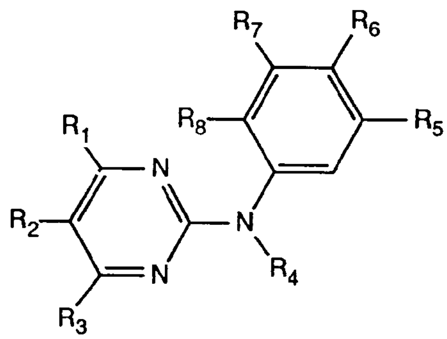

(I)

worin $R_1$ Wasserstoff oder $C_1$-$C_3$-Alkyl bedeutet, $R_2$ Wasserstoff oder $C_1$-$C_3$-Alkyl bedeutet, $R_3$ 2-Pyridyl, 3-Pyridyl, 4-Pyridyl, 2-Methyl-3-pyridyl, 4-Methyl-3-pyridyl, 2-Furyl, 5-Methyl-2-furyl, 2,5-Dimethyl-3-furyl, 2-Thienyl, 3-Thienyl, 5-Methyl-2-thienyl, 2-Phenothiazinyl, 4-Pyrazinyl, 2-Benzofuryl, N-Oxido-2-pyridyl, N-Oxido-3-pyridyl, N-Oxido-4-pyridyl, 1H-Indol-2-yl, 1H-Indol-3-yl, 1-Methyl-1H-pyrrol-2-yl, 4-Quinolinyl, 1-Methyl-pyridinium-4-yl-iodid, Dimethylamino-phenyl oder N-Acetyl-N-methyl-amino- phenyl bedeutet, $R_4$ Wasserstoff, $C_1$-$C_3$-Alkyl, den Rest $-CO-CO-O-C_2H_5$ oder N,N-Dimethylaminoethyl bedeutet, mindestens einer der Reste $R_5$, $R_6$, $R_7$ und $R_8$ $C_1$-$C_6$-Alkyl, $C_1$-$C_3$-Alkoxy, Chlor, Brom, Iod, Trifluormethyl, Hydroxy, Phenyl, Amino, Mono- ($C_1$-$C_3$-alkyl)-amino, Di-($C_1$-$C_3$-alkyl)-amino, $C_2$-$C_4$-Alkanoyl, Propenyloxy, Carboxy, Carboxy-methoxy, Ethoxycarbonyl-methoxy, Sulfanilamido, N,N-Di-($C_1$-$C_3$-alkyl)-sulfanilamido, N-Methyl-piperazinyl, Piperidinyl, 1H-Imidazol-1-yl, 1H-Triazol-1-yl, 1H-Benzimidazol-2-yl, 1-Naphthyl, Cyclopentyl, 3,4-Dimethyl-benzyl oder einen Rest einer der Formeln:
$-CO_2R$, $-NH-C(=O)-R$, $-N(R)-C(=O)-R$, $-O-(CH_2)_n-N(R)-R$, $-C(=O)-NH-(CH_2)_n-N(R)-R$, $-CH(CH_3)-NH-CHO$, $-C(CH_3)=N-OH$, $-C(CH_3)=N-O-CH_3$, $-C(CH_3)-NH_2$, $-NH-CH_2-C(=O)-N(R)-R$,

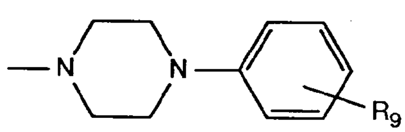,

$-(CH_2)_m-R_{10}$, $-X-(CH_2)_m-R_{10}$ oder

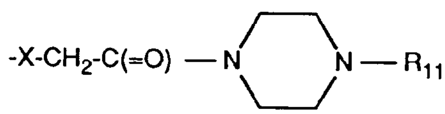

bedeutet, worin R für $C_1$-$C_3$-Alkyl steht, X für Sauerstoff oder Schwefel steht, m für 1, 2 oder 3 steht, n für 2 oder 3 steht, $R_9$ für Wasserstoff, $C_1$-$C_3$-Alkyl, $C_1$-$C_3$-Alkoxy, Chlor, Brom, Iod oder Trifluormethyl steht, $R_{10}$ für 1H-Imidazol-1-yl oder Morpholinyl steht und $R_{11}$ für $C_1$-$C_3$-Alkyl oder unsubstituiertes oder durch $C_1$-$C_3$-Alkyl, Halogen oder Trifluormethyl monosubstituiertes Phenyl steht, und die übrigen der Reste $R_5$, $R_6$, $R_7$ und $R_8$ Wasserstoff bedeuten, oder von pharmazeutisch verwendbaren Salzen von solchen Verbindungen mit mindestens einer salzbildenden Gruppe zur Herstellung von pharmazeutischen Präparaten zur Anwendung als Antitumormittel in Warmblütern einschliesslich des Menschen.

5. Verwendung nach einem der Ansprüche 1-4, worin in der Verbindung der Formel I mindestens zwei der Reste $R_5$, $R_6$ und $R_8$ je für Wasserstoff stehen.

6. Verwendung nach einem der Ansprüche 1-4, worin in der Verbindung der Formel I $R_5$, $R_8$ und $R_8$ je für Wasserstoff stehen.

7. Verwendung nach einem der Ansprüche 1-4, worin in der Verbindung der Formel I $R_1$, $R_2$, $R_4$, $R_8$, $R_6$ und $R_8$ je für Wasserstoff stehen, $R_3$ 2-Pyridyl, 3-Pyridyl, 4-Pyridyl, 2-Methyl-3-pyridyl, 4-Methyl-3-pyridyl, 2-Furyl, 3-Methyl-2-furyl, 2,5-Dimethyl-3-furyl, 2-Thienyl, 3-Thienyl, 5-Methyl-2-thienyl, 2-Phenothiazinyl, 4-Pyrazinyl, 2-Benzofuryl, N-Oxido-2-pyridyl, N-Oxido-3-pyridyl, N-Oxido-4-pyridyl, 1H-Indol-2-yl, 1H-Indol-3-yl, 1-Methyl- 1H-pyrrol-2-yl, 4-Quinolinyl, 1-Methyl-pyridinium-4-yl-iodid, Dimethylamino-phenyl oder N-Acetyl-N-methyl-amino-phenyl bedeutet, und $R_7$ $C_1$-$C_6$-Alkyl, $C_1$-$C_3$-Alkoxy, Chlor, Brom, Iod, Trifluormethyl, Hydroxy, Phenyl, Amino, Mono-($C_1$-$C_3$-alkyl)-amino, Di-($C_1$-$C_3$-alkyl)-amino, $C_2$-$C_4$-Alkanoyl, Propenyloxy, Carboxy, Carboxy-methoxy, Ethoxycarbonyl-methoxy, Sulfanilamido, N,N-Di-($C_1$-$C_3$-alkyl)-sulfanilamido, N-Methyl-piperazinyl, Piperidinyl, 1H-Imidazol-1-yl, 1H-Triazol-1-yl, 1H-Benzimidazol-2-yl, 1-Naphthyl, Cyclopentyl, 3,4-Dimethyl-benzyl oder einen Rest einer der Formeln:
$-CO_2R$, -NH-C(=O)-R, -N(R)-C(=O)-R, $-O-(CH_2)_n-N(R)-R$, $C(=O)-NH-(CH_2)_n-N(R)-R$, $-CH(CH_3)-NH-CHO$, $-C(CH_3)=N-OH$, $-C(CH_3)=N-O-CH_3$, $-C(CH_3)-NH_2$, $-NH-CH_2-C(=O)-N(R)-R$,

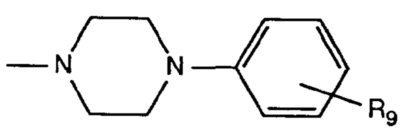
,

$-(CH_2)_m-R_{10}$, $-X-(CH_2)_m-R_{10}$ oder

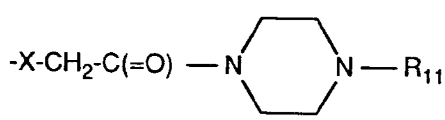

bedeutet, worin R für $C_1$-$C_3$-Alkyl steht, X für Sauerstoff oder Schwefel steht, in für 1, 2 oder 3 steht, n für 2 oder 3 steht, $R_9$ für Wasserstoff, $C_1$-$C_3$-Alkyl, $C_1$-$C_3$-Alkoxy, Chlor, Brom, Iod oder Trifluormethyl steht, $R_{10}$ für 1H-Imidazol-1-yl oder Morpholinyl steht und $R_{11}$ für $C_1$-$C_3$-Alkyl oder unsubstituiertes oder durch $C_1$-$C_3$-Alkyl, Halogen oder Trifluor methyl monosubstituiertes Phenyl steht.

8. Verwendung nach einem der Ansprüche 1-4, worin in der Verbindung der Formel I $R_1$, $R_2$, $R_4$, $R_8$, $R_6$ und $R_8$ je für Wasserstoff stehen, $R_3$ 3-Pyridyl bedeutet und $R_7$ 1H-Imidazol-1-yl, Amino, Trifluormethyl, Chlor oder einen Rest der Formel $-CO_2R$ oder $-C(=O)-NH-(CH_2)_n-N(R)-R$ bedeutet, worin R je für Wasserstoff oder Methyl und n für 3 stehen.

9. Verwendung nach einem der Ansprüche 1-4, worin man als Verbindung der Formel I N-(3-1H-Imidazol-1-yl-phenyl)-4-(3-pyndyl)-2-pyrimidinamin oder ein pharmazeutisch verwendbares Salz davon einsetzt.

10. Verwendung nach einem der Ansprüche 1-4, worin man als Verbindung der Formel I N-(3-Trifluorimethyl-phenyl)4-(3-pyridyl)-2-pyrimidulamin oder ein pharmazeutisch verwendbares Salz davon einsetzt.

**11.** Verwendung nach einem der Ansprüche 1-4, worin man als Verbindung der Formel I N-(3-Chlor-phenyl)4-(3-pyridyl)-2-pyrimidinamin oder ein pharmazeutisch verwendbares Salz davon einsetzt.

**12.** Verwendung nach einem der Ansprüche 1-4, worin man als Verbindung der Formel I N-(3-Amino-phenyl)-4-(3-pyridyl)-2-pyrimidinamin oder ein pharmazeutisch verwendbares Salz davon einsetzt.

**13.** Verwendung nach einem der Ansprüche 1-4, worin man als Verbindung der Formel I N-(3-Methoxycarbonyl-phenyl)-4-(3-pyridyl)-2-pyrimidinamin oder ein pharmazeutisch verwendbares Salz davon einsetzt.

**14.** Verwendung nach einem der Ansprüche 1-4, worin man als Verbindung der Formel I N-(3-[3-Amino-propylamino-carbonyl]-phenyl)-4-(3-pyridyl)-2-pyrimidinamin oder ein pharmazeutisch verwendbares Salz davon einsetzt.

Europäisches Patentamt

# EUROPÄISCHER TEILRECHERCHENBERICHT

der nach Regel 45 des Europäischen Patentübereinkommens für das weitere Verfahren als europäischer Recherchenbericht gilt

Nummer der Anmeldung: EP 93 81 0595

## EINSCHLÄGIGE DOKUMENTE

| Kategorie | Kennzeichnung des Dokuments mit Angabe, soweit erforderlich der maßgeblichen Teile | Betrifft Anspruch | KLASSIFIKATION DER ANMELDUNG (Int.Cl.5) |
|---|---|---|---|
| D,A | EP-A-0 233 461 (AMERICAN CYANAMID COMP.) 26. August 1987<br>* das ganze Dokument * | 1-14 | A61K31/505 |
| A | EP-A-0 453 731 (AMERICAN CYANAMID COMPANY) 30. Oktober 1991<br>* das ganze Dokument * | 1-14 | |
| A | EP-A-0 164 204 (FISONS) 11. Dezember 1985<br>* das ganze Dokument * | 1-14 | |

RECHERCHIERTE SACHGEBIETE (Int.Cl.5): A61K

## UNVOLLSTÄNDIGE RECHERCHE

Nach Auffassung der Recherchenabteilung entspricht die vorliegende europäische Patentanmeldung den Vorschriften des Europäischen patentübereinkommens so wenig, daß es nicht möglich ist, auf der Grundlage einiger Patentansprüche sinnvolle Ermittlungen über den Stand der Technik durchzuführen.
Vollständig recherchierte Patentansprüche:
Unvollständig recherchierte Patentansprüche:
Nicht recherchierte Patentansprüche:
Grund für die Beschränkung der Recherche:

Siehe Ergänzungsblatt C

| Recherchenort | Abschlußdatum der Recherche | Prüfer |
|---|---|---|
| DEN HAAG | 27. Dezember 1993 | Krautbauer, B |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C09)

EP 93 81 0595 -C-

UNVOLLSTÄNDIGE RECHERCHE

Vollständig recherchierte Patentansprüche : 9-14
Unvollständig recherchierte Patentansprüche : 1-8

Grund : Auf grund der Vielzahl an theoretisch denkbaren Substanzen, die sich aus der verwendeten Markush-Formel ergeben, musste die Recherche auf die in den Ansprüchen explizit genannten Substanzen sowie auf das allgemeine erfinderische Konzept beschränkt werden.